# EUROPEAN PATENT APPLICATION

(11) **EP 2 172 152 A1**
(43) Date of publication of application: **07.04.2010**
(21) Application number: 08165956.7
(22) Date of filing: 06.10.2008
(51) Int. Cl.: A61B 5/0478

(54) **Electrode for medical applications.**

(71) Applicant: Nederlandse Organisatie voor toegepast-natuurwetenschappelijk onderzoek TNO, 2628 VK Delft (NL)
(72) Inventor: van Grinsven, BArt, 2328 VK Delft (NL); Hoppenbrouwers, Marcus Benedictus, 5611 JX Eindhoven (NL)
(74) Representative: Hatzmann, Martin

(57) **Abstract**

Electrode for medical applications, comprising a base part (1), which is connectable to an electric lead or other conductor, the base part including a multitude of mainly truncated teeth (3), each of them comprising, at its mainly truncated surface a number of microspikes (5), i.e. small pointed bodies. The electrode can be manufactured by injection molding and subsequent metallization, e.g. made from PMMA and subsequently gold-plated.

## Description

The invention refers to an electrode for medical applications.

Skin electrodes are widely used in the medical and biomedical world. Electrodes are commonly used for measuring biopotential and possibly for electrical stimulation. The most important measured potentials are the electrocardiogram, electromyelogram, electroencephalogram, electrooculogram and the nerve conduction of action potentials. The characteristics of the electrodes used are of great importance, because they represent the starting point of the measurement. If incorrect electrodes are selected, artifacts or misleading data are inevitable.

A major disadvantage of the presently used standard electrodes is the extensive skin preparation required and frequently the use of an electrolytic gel. Long preparation times and long stabilization times are necessary before the electrolytic gel has diffused into the skin; besides this, the gel has to be removed after the measurement. All of this results in a lack of comfort for both investigator and subject. Studies aimed at reducing the problems of the use of standard electrodes have already led to the development of new types of electrodes for measuring biopotentials, the "Nasicon ceramic electrodes," "Chip amplified dry electrodes," and electrodes that are sold under the trade name "Zipprep" [see: Non-Polarisable Dry Electrode Based on NASICON Ceramic, Ch. Gondran et al., Medical & Biological Engineering & Computing, May 1995; An Active, Micro fabricated, Scalp Electrode-Array for EEG Recording, Babak Alizadeh Taheri, Rosemary L. Smith and Robert T. Knight, Sensors and Actuators A54, Elsevier Science S.A., 1996; and Low-cost Active Electrode Improves the Resolution in Biopotential Recordings, Alexander C. Metting VanRijn, Anthony P. Kuiper, Taco E. Dankers and Cees A. Grimpergen, 18th Int. Conf. of the IEEE Engineering in Medicine and Biology Society, Amsterdam, 1996.]

Biopotentials originate from the electrochemical activity of polarizable active cells. These cells are a component of nervous tissue, muscular tissue or glandular tissue. In the human body, such cells are surrounded by body fluids with a high Cl⁻ ion concentration. A polarizable cell behaves as a constant source of current when it is stimulated and creates an ionic current in the body fluid. This current induces electrical biopotentials in the body. The amplitude of these biopotentials decreases with increasing distance from the active cell. The detection of biopotentials is therefore closely related to the ionic current created by the active cells. Modem electronics (e.g. amplifiers) require an electronic current. One electrode functions as a transducer which transforms ionic current into an electrical current. This transformation is possible if an electrochemical electrode-electrolyte interface is formed. In this case, the body fluid is the electrolyte, and a metallic biopotential is the chemical electrode.

When the EEG is measured, a number of barriers exist between the electrode and the voltage source, which interfere with the measurement of this potential. A first obvious barrier is the hair usually present on the skin of the head (scalp). Hair has insulating characteristics and is an undesired barrier between source and electrode. At the present time, it is advisable to shave the hair off before a measurement can be begun. This frequently leads to long preparation times and unwillingness of the participants (from the aesthetic standpoint).

The anatomy of skin is another important point. The skin has a layered architecture. The outermost layer of skin, the *stratum corneum,* is a barrier having electrically insulating characteristics. This layer renews itself constantly and consists of dead skin cells. The *stratum germinativum* is the layer where cell division and cell growth takes place and the cells are positioned in the direction of the *stratum corneum.* The *stratum germinativum* consists of living cells which in turn consist of liquid. This skin layer is electrically conductive tissue comparable with the conductivity of an electrolyte. The dermis is located under the *stratum germinativum.* The dermis contains blood vessels, nerve fibers, sweat glands and hair follicles. The dermis is also electrically conducting, and is where the sensation of pain originates

When an electrode is placed on unprepared skin, very high skin-electrode impedance will be the result due to the fact that no direct electrochemical electrode-electrolyte interaction with the body fluids is achieved. Therefore, the conventional biopotential electrodes require skin preparation before the electrode can be positioned. The two most commonly used preparation methods are the shaving (off) of the *stratum corneum* and the use of electrolytic gel between the skin and the electrode. The purpose of shaving is to reduce the thickness of the stratum corneum. Shaving off this layer completely, however, is painful and not recommended. Electrolytic gel with a high concentration of Na⁺- and Cl⁻ ions can be applied to the *stratum corneum.* The gel diffuses into the layer and improves the conductivity. The combination of the above-noted methods reduces the electrode-skin impedance by creating an electrode-electrolyte layer. Unfortunately, the disadvantages of these methods are very clear: long preparation times, long stabilization times (diffusion of the gel into the skin), long after-care times, persistent discomfort. Biopotential electrodes that are not based on an electrochemical electrode-electrolyte layer have already been described. These electrodes are active (signal amplification on the electrode itself) and make use of capacitive detection methods. The disadvantage of these electrodes is their complexity and high costs.

One aim of the present invention is to provide an electrode for medical applications which has better electrical properties. Another aim is to provide such an electrode which requires less preparation and care. Yet another aim is to provide an electrode which does not need the patient's hair to be shaven off. Still another aim is to provide an electrode which is simple, reliable and rather cheap because it can be manufactured by e.g. injection molding and metalizing.

According to the invention, the electrode comprises a base part, which is connectable to an electric lead or other conductor, and which includes a multitude of mainly truncated teeth, each of them comprises, at its mainly truncated surface a number of microspikes, i.e. small pointed bodies.

Preferably, the height of the teeth is between 1 and 5 mm, more preferably between 1.5 and 2 mm. The height of the microspikes preferably is between 10 and 20 µm, more preferably between 15 and 17 µm.

The electrode according to the invention preferably is manufactured by injection molding, more preferably of PMMA, and subsequent metallization, more preferably gold-plating.

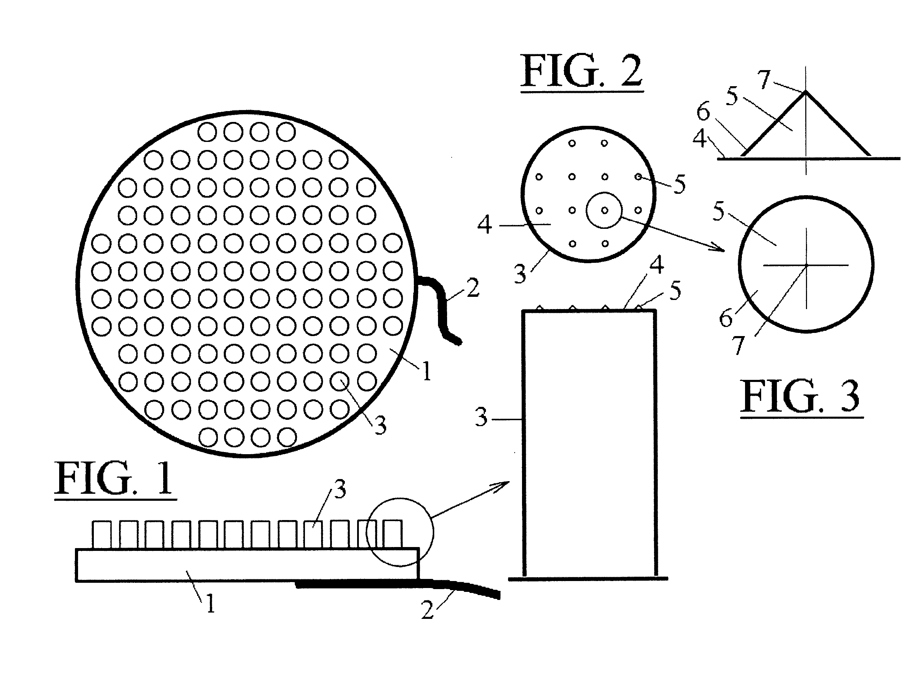

- Figure 1: shows an exemplary embodiment of the electrode according to the invention, in tow views;
- Figure 2: shows, in two views, the electrode teeth more in detail;
- Figure 3: shows, in two views, the micro spikes more in detail.

The electrode shown in the figures comprises a base part 1, which is connectable to an electric lead or other conductor 2, and which includes a multitude of mainly truncated teeth 3. Each of those teeth 3 comprise, at its truncated surface 4 a number of microspikes 5, i.e. small pointed bodies, including a base 6 and a point 7.

The height of the teeth 3 preferably is between 1.5 and 2 mm, their diameter between 0.8 and 1.5 mm and their density between 10 and 30 cm⁻¹. The height of the microspikes 5 preferably is between 15 and 17 µm, the width or diameter of their base 6 between 15 and 25 µm and their density being between 100 and 200 mm⁻¹.

The electrode, including its base part 1, its teeth 3 and its microspikes 5 preferably is manufactured by injection molding of PMMA, and subsequent metallization, preferably gold-plating.

The teeth structure of about 1.5 mm height is placed in a selected density over the entire surface of the electrode. These teeth 3 give the electrode a "comb" effect. Hairs are guided between the teeth, thus overcoming the first barrier, and the electrode starts directly measuring on the patient's skin or scalp without a foregoing preparation time. Now the electrode encounters the second barrier, which is overcome by providing each tooth of the electrode with a structure of "microspikes". This micro spike structure pierces the *stratum corneum* and is supposed to penetrate into the electrically conducting *germinativum.* In this way the high impedance of the *stratum corneum* is circumvented. Certainly, it must be assured that the teeth do not extend deeper than the *stratum germinativum,* which contains nerves and blood vessels to prevent a sensation of pain. The thickness of the *stratum corneum* is approximately 15 µm, the thickness of the *stratum germinativum* 50 µm. The teeth 3 will be maximally 17 µm long. In this way, the *stratum corneum* is pierced and the *stratum germinativum* penetrated. In this way, a painless electrode-electrolyte layer is realized in the *stratum germinativum,* and ionic current is transformed, induced by repolarizable cells, into electric current. Besides this, the (Netherlands) government has drawn a limit at 18 µm. If more than 18 µm of skin is penetrated, that will be considered as a subcutaneous treatment.

## Claims

1. Electrode for medical applications, comprising a base part (1), which is connectable to an electric lead or other conductor, the base part including a multitude of mainly truncated teeth (3), each of them comprising, at its mainly truncated surface a number of microspikes (5), i.e. small pointed bodies.

2. Electrode according to claim 1, the height of the teeth being between 1 and 5 mm.

3. Electrode according to claim 2, the height of the teeth being between 1.5 and 2 mm.

4. Electrode according to any of claims 2 - 3, the diameter of the teeth being between 0.8 and 1.5 mm.

5. Electrode according to any of claims 2 - 4, the density of the teeth being between 10 and 30 cm⁻¹

6. Electrode according to any preceding claim, the height of the microspikes being between 10 and 20 µm.

7. Electrode according to claim 6, the height of the microspikes being between 15 and 17 µm.

8. Electrode according any of claims 6 - 7, the width or diameter of the base (6) of the microspikes being between 15 and 25 µm.

9. Electrode according to any of claims 6 - 8, the density of the microspikes being between 100 and 200 mm⁻¹

10. Electrode according to any preceding claim, being manufactured by injection molding and subsequent metallization.

11. Electrode according to claim 10, made from PMMA (Polymethyl methacrylate).

12. Electrode according to claim 10, being gold-plated.
